# FASCICULE DE BREVET EUROPEEN

(11) **EP 1 313 877 B1**
(45) Date de publication et mention de la délivrance du brevet: **30.12.2009**
(21) Numéro de dépôt: 01963106.8
(22) Date de dépôt: 17.08.2001
(51) Int. Cl.: C12Q 1/56, G01N 33/86

(54) **METHODE DE DOSAGE DE LA FIBRINE SOLUBLE**
VERFAHREN ZUR BESTIMMUNG VON LÖSLICHEM FIBRIN
ASSAY FOR SOLUBLE FIBRIN

(30) Priorité: 28.08.2000 FR 0010999
(43) Date de publication de la demande: 28.05.2003
(73) Titulaire: Diagnostica Stago, 92600 Asnières (FR); ASSISTANCE PUBLIQUE-HOPITAUX DE PARIS, 75100 Paris (FR)
(72) Inventeur: MIRSHAHI, Bibi, Shah, Soltan, F-94800 Villejuif (FR); SORIA, Jeannette, F-95150 Taverny (FR)
(74) Mandataire: Desaix, Anne
(86) Numéro de dépôt international: PCT/FR2001/002628
(87) Numéro de publication internationale: WO 2002/018628

(56) Documents cités:
- EP-A- 0 347 933
- EP-A- 0 576 038
- US-A- 5 114 845
- US-A- 5 453 359
- US-A- 6 048 684
- DATABASE BIOSIS [en ligne] BIOSCIENCES INFORMATION SERVICE, PHILADELPHIA, PA, US; WIMAN B ET AL: "DETERMINATION OF SOLUBLE FIBRIN IN PLASMA BY A RAPID AND QUANTITATIVE SPECTROPHOTOMETRIC ASSAY." retrieved from STN Database accession no. 1986:316748 XP002171140 & THROMB HAEMOSTASIS, (1986) 55 (2), 189-193.,

## Description

La présente invention a trait à une méthode de dosage de la fibrine soluble par génération de produits de dégradation spécifiques dans un échantillon sanguin.

Lors de l'activation de la coagulation, il y a génération de thrombine qui entraîne la formation de dépôts de fibrine et de formation de fibrine soluble.

En effet, la thrombine va détacher de la molécule de fibrinogène le fibrinopeptide A entraînant la génération de monomères de fibrine sur lesquels les sites de polymérisation "A" qui étaient, masqués dans le fibrinogène sont démasqués, entraînant de ce fait une interaction entre les sites "A" d'une molécule de monomère de fibrine avec les sites "a" accessibles aussi bien sur le fibrinogène que sur la fibrine. Dans un 2^{ème} temps, il y a libération de fibrinopeptide B, entraînant un démasquage des sites de polymérisation "B" entraînant une interaction entre les sites "B" d'une molécule de monomère de fibrine avec les sites "b" accessibles aussi bien sur le fibrinogène que sur les monomères de fibrine.

Lorsque la quantité de thrombine est très importante (tests in vitro), tout le fibrinogène est transformé en monomères de fibrine qui se polymérisent par suite de l'interaction des sites « A » et « a » d'une part, « B » et « b » d'autre part, pour donner le caillot de fibrine. En revanche, in vivo, la génération de thrombine est beaucoup moins importante. La génération de monomères de fibrine est ainsi beaucoup moins explosive et de ce fait une partie des monomères de fibrine vont polymériser pour donner de la fibrine (thrombus) et une autre partie va réagir avec du fibrinogène où les sites a et b sont accessibles ou avec des produits de dégradation du fibrinogène, pour donner de la fibrine soluble dans laquelle des monomères de fibrine sont associés avec du fibrinogène.

La détermination de la fibrine soluble a pour but d'étudier s'il existe une activation de la coagulation chez un patient, la présence de fibrine soluble dans le sang, en particulier dans le plasma, étant le témoin de cette activation.

Cette détermination est un complément nécessaire au dosage de D-dimères formés par la fibrinolyse de la fibrine constituant le thrombus, qui est également un marqueur du processus d'activation de la coagulation. En effet, la teneur plasmatique en D-dimères est augmentée tant que le caillot se dégrade IN VIVO. De ce fait, si le thrombus est présent et est en train de se dégrader, le taux de D-dimères sera augmenté, que la coagulation persiste ou soit arrêtée ; celui de fibrine soluble en revanche, n'augmentera plus si la coagulation est arrêtée et en revanche augmentera si la coagulation persiste.

La mesure spécifique de la teneur plasmatique en fibrine soluble par rapport à la teneur en D-dimères permet donc :
1. de déterminer si un processus de coagulation est présent chez un patient au moment où est effectué le prélèvement,
2. d'évaluer la balance coagulo-lytique. En effet, le taux de D-dimères de base est le reflet de la dégradation du thrombus IN VIVO ; le taux de D-dimères obtenu après addition d'agent thrombolytique dans le plasma représente la somme des D-dimères de base et celle provenant de la dégradation de la fibrine circulante (ou fibrine soluble).

Parmi les méthodes de dosage de la fibrine soluble les plus anciennes, on peut citer le test à l'éthanol (1, 2, 9) ou celui au sulfate de protamine (3, 4, 5, 7). Toutefois, ces tests sont peu spécifiques (9, 10) et peu sensibles (10). De plus, des taux élevés de fibrinogène (> 5 g/l) perturbent les résultats obtenus avec les tests à l'éthanol et au sulfate de protamine. Enfin, les résultats des tests au sulfate de protamine peuvent être difficiles à interpréter (6, 8).

Un autre type de détection de la fibrine soluble est basé sur une technique d'hémagglutination de globules rouges sensibilisés avec des monomères de fibrine selon la méthode de Largo (11, 12). Ce type de test est par exemple commercialisé par la société Diagnostica Stago sous la dénomination de FS Test.

Cette technique de réalisation, bien que très aisée peut cependant manquer parfois de sensibilité et se prête surtout au diagnostic de coagulations intra-vasculaires disséminées. Elle ne permet pas, en revanche, de déceler des teneurs plus faibles en fibrine soluble (thromboses localisées, exploration de l'efficacité d'une thérapeutique anti-coagulante).

A l'heure actuelle, les autres techniques de dosage de la fibrine soluble sont basées sur l'utilisation d'anticorps monoclonaux détectant des épitopes démasqués dans la fibrine et masqués dans le fibrinogène ou les produits de dégradation de la fibrine ou du fibrinogène (13-14). Toutefois, les dosages directs par l'utilisation d'anticorps monoclonaux conduisent à des taux de fibrine soluble qui varient selon l'anticorps commercial utilisé.

Les auteurs de la présente invention proposent une approche différente de celles préconisées dans l'état de l'art, particulièrement simple et rapide pour apprécier la balance coagulo-lytique d'un patient. Celle-ci possède une meilleure sensibilité que la méthode par hémagglutination décrite ci-dessus. Elle présente l'avantage, par rapport aux tests utilisant des anticorps monoclonaux, de détecter de la même manière la fibrine circulante (soluble) et celle déjà dégradée IN VIVO, permettant ainsi d'avoir une évaluation très exacte de la balance coagulo-lytique.

Selon la méthode de la présente invention, la fibrine soluble présente dans un échantillon est mesurée après génération de produits de dégradation de la fibrine soluble, lors de l'incubation de l'échantillon avec un activateur du plasminogène ayant une forte spécificité pour la fibrine (PA-Fb sp). Ainsi, la différence entre le taux de produits de dégradation obtenu après dégradation de la fibrine soluble par le PA-Fb sp et celui des produits de dégradation de la fibrine de base, mesuré avant la mise en contact de l'échantillon avec le PA-Fbsp, permet de mesurer la teneur plasmatique en fibrine soluble dans le prélèvement.

L'invention a donc pour objet une méthode de dosage de la fibrine soluble dans un échantillon biologique dans laquelle on met ledit échantillon en présence d'un activateur du plasminogène de forte affinité pour la fibrine (PA-Fb sp) et l'on mesure la teneur de l'échantillon en fibrine soluble en mesurant la différence entre le taux de produits de dégradation obtenus après dégradation de la fibrine soluble par le PA-Fb sp et le taux de base des produits de dégradation de la fibrine déterminé avant la mise en présence de l'échantillon avec le PA-Fb sp.

La méthode de dosage de la fibrine soluble selon l'invention dans un échantillon biologique, comprend les étapes de :
- dosage des produits de dégradation de la fibrine contenus dans l'échantillon de plasma prélevé,
- mise en contact de l'échantillon sanguin avec un activateur de plasminogène ayant une forte spécificité pour la fibrine (Pa-Fb sp) dans des conditions permettant à la fibrine soluble contenue dans l'échantillon, d'être dégradée en ses produits de dégradation, les conditions d'utilisation de l'activateur étant sélectionnées pour éviter la dégradation du fibrinogène plasmatique circulant,
- dosage des produits de dégradation de la fibrine dans l'échantillon incubé avec le Pa-Fbsp
- détermination de la fibrine soluble correspondant à la différence entre la teneur en produits de dégradation de la fibrine évaluée après incubation avec le PA-Fb sp et la teneur en produits de dégradation de la fibrine, évaluée dans l'échantillon non traité.

Le réactif utilisé pour doser les produits de dégradation est choisi pour mesurer un groupe donné de produits de dégradation. Par exemple, on utilise des anticorps de spécificité déterminée vis à vis d'un type particulier de produits de dégradation.

L'échantillon biologique est de préférence un liquide biologique, par exemple un échantillon de sang ou de plasma, ou encore un liquide de ponction.

Est décrite également ici l'utilisation d'un activateur du plasminogène de forte affinité pour la fibrine (i.e. qui n'active le plasminogène qu'au sein de la fibrine) dans une méthode de dosage de la fibrine soluble par génération de produits de dégradation spécifiques. Elle vise également un kit pour la mise en oeuvre de la méthode citée ci-dessus.

On connaît de nombreux composés activateurs du plasminogène. Certains toutefois dégradent aussi bien le fibrinogène que la fibrine, tels par exemple la streptokinase et l'urokinase (15). Ces composés ne sont pas adaptés à la méthode de l'invention car ils conduisent à une dégradation du fibrinogène donnant lieu à des produits de dégradation du fibrinogène qui interfèrent avec ceux résultant de la dégradation de la fibrine.

Un second groupe d'activateurs du plasminogène est constitué par des composés décrits comme présentant une forte spécificité pour dégrader la fibrine, par rapport au fibrinogène. La méthode de l'invention utilise avantageusement la spécificité de ce second groupe de composés pour sa mise en oeuvre.

Différents composés présentant une telle spécificité (PA Fb sp) ont été décrits dans la littérature. On connaît par exemple :
◆ l'activateur tissulaire du plasminogène (ou t-PA) ou ses dérivés comme, par exemple, le TNK-tPA qui est un mutant du t-PA qui a une très grande spécificité pour la fibrine (16) ;
◆ l'activateur provenant de la salive de Desmodus rotundus (bat-tPA ou vPA = Vampire bar salivary plasminogen activator) ou ses dérivés :
   DSPAs = Desmodus rotundus salivary PAs, FEKP = DSPA alpha 1 et alpha 2, EKP = DSPA beta, KP = DSPA gamma, (17).
◆ la Staphylokinase, (SAK), polypeptide sécrété par le Staphylococcus aureus (18-19) ou un de ses mutants (20).

La méthode selon la présente invention est préférentiellement réalisée sur un échantillon de plasma. Le taux de fibrine soluble, est déterminé par la mesure des produits de dégradation résultant de l'action du PA Fb sp. La méthode est validée si besoin grâce à l'utilisation d'un témoin positif obtenu à partir d'un plasma normal traité avec des traces de thrombine de façon à induire une activation de la coagulation responsable de la génération de fibrine soluble, sans aboutir toutefois à la formation d'un caillot.

Pour l'obtention du plasma contrôle positif, le plasma est d'abord incubé avec de la thrombine ou autre activateur de la coagulation pendant un temps déterminé. Le processus de coagulation qui a été ainsi déclenché est ensuite bloqué par l'addition d'un inhibiteur de l'activateur utilisé pour éviter que la réaction ne se poursuive. Dans le cas où cet activateur est la thrombine, on utilise par exemple l'hirudine ou l'héparine en tant qu'inhibiteur.

Les temps d'incubation du plasma et les concentrations en activateur de la coagulation et en inhibiteur pour le blocage sont avantageusement déterminés de façon à obtenir toutes les étapes d'activation de la coagulation précédant le début de la formation du caillot.

L'incubation en présence d'activateur de la coagulation (thrombine) est de préférence réalisée pendant un temps d'incubation de 2 minutes, à température ambiante. L'inhibiteur est ensuite ajouté en large excès pour être sûr de bloquer la coagulation.
◆ S'il s'agit de l'hirudine, celle-ci est avantageusement utilisée à une concentration finale de 100 µg/ml pour une concentration finale en thrombine de 0,18 U/ml.
◆ S'il s'agit d'héparine, celle-ci est utilisée à 500 U/ml en concentration finale lorsque la concentration finale en thrombine utilisée est de 0,18U/ml.

L'évaluation de la fibrine soluble selon la présente invention met en oeuvre une première étape de dégradation de la fibrine soluble par le PA Fb sp, suivie d'une mesure des produits de dégradation spécifiques résultant de l'action du PA Fb sp.

Il est indispensable que les résultats de la méthode selon l'invention puissent être obtenus le plus rapidement possible, tout en étant représentatifs de la quantité de fibrine soluble présente dans l'échantillon. Pour ce faire, les conditions d'utilisation du PA Fb sp doivent être déterminées de façon à ce que la dégradation de la fibrine soluble soit rapide et qu'elle ne s'accompagne pas d'une dégradation "contaminante" du fibrinogène plasmatique circulant, donnant lieu à des produits de dégradation interférant avec ceux provenant de la fibrine soluble dans le dosage.

Les doses de PA Fb sp sont ainsi choisies de façon à induire l'augmentation du taux de produits de dégradation de la fibrine la plus importante dans les témoins positifs et une augmentation pratiquement nulle dans les témoins négatifs (i.e. n'ayant pas subi de traitement par un activateur de la coagulation).

Différents activateurs de la fibrinolyse permettant la dégradation spécifique de la fibrine peuvent être utilisés dans le cadre de la présente invention. Avantageusement, le PA Fb sp est choisi au sein du groupe constitué par les activateurs cités précédemment, à savoir: le tPA ou ses dérivés, le VPA ou ses dérivés, la staphylokinase ou un de ses mutants. On utilise de préférence le tPA ou la Staphylokinase, et plus préférentiellement, le tPA.

Dans des conditions d'incubation des échantillons de 15 minutes à 37° C, la concentration finale de staphylokinase utilisée est comprise entre 1 et 12 µg/ml. La concentration finale retenue est de 10 µg/ml. Le temps d'incubation peut être modifié et sa variation sera déterminée en fonction de la nature et de la concentration de la PaFbsp utilisée.

Le tPA est avantageusement utilisé dans une gamme de concentrations finales comprise entre 1 et 2,5 µg/ml. Préférentiellement, le tPA est utilisé à 2 µg/ml.

Il existe différents produits de dégradation de la fibrine soluble qui peuvent être spécifiquement détectés. Selon un mode de réalisation préféré, on mesure le taux de D-dimères résultant de l'action du PA Fb sp sur la fibrine soluble, i.e. on évalue la concentration en D-dimères résultant de l'action du PA Fb sp sur la fibrine soluble (D-dimères après action de PA Fb sp - D-dimères de base avant l'action de PA Fb sp).

Les D-Dimères résultant de la dégradation de la fibrine soluble en présence de PA Fb sp, peuvent être dosés selon toute technique courante de dosage d'analyte telles que par exemple des méthodes de type ELISA, des méthodes sensibles par agglutination de billes de latex, des méthodes d'immunochromatographie, etc. Parmi les différents tests de dosage du D-Dimère disponibles dans le commerce, on peut citer par exemple l'ASSERACHROM D-Di ou le STA LIATEST D-Di, tous deux commercialisés par Diagnostica Stago. Toutefois, dans le cadre de la présente invention, les conditions d'utilisation du test ELISA de l'ASSERACHROM D-Di ont été avantageusement modifiées pour raccourcir le test (15 min. d'incubation avec l'anticorps immobilisé et 15 minutes avec l'anticorps marqué à la peroxydase).

Outre le fragment DD/E, il existe d'autres produits de dégradation de la fibrine tels que les complexes YD/DY, YD/DXD qui peuvent être évalués.

Comme cela est illustré dans les exemples ci-après (voir exemple n° 3), la méthode de l'invention peut être réalisée chez des malades présentant une activation de la coagulation soit avant le démarrage d'une thérapie, soit en cours de traitement anti-coagulant et soit après l'arrêt de la thérapie anti-coagulante. Elle permet ainsi d'apprécier non seulement l'évolution d'un processus d'activation de la coagulation, en particulier dans le cadre du diagnostic d'activation de la coagulation, mais également l'efficacité d'une thérapeutique anti-coagulante.

Selon un autre aspect, il est ici décrit un kit (ensemble) de dosage de la fibrine soluble dans un échantillon, caractérisé en ce qu'il comprend :
◆ un contrôle positif pour la présence de fibrine soluble obtenu selon le protocole décrit précédemment,
◆ un contrôle négatif constitué d'un plasma témoin,
◆ du PA-Fb sp en quantité individuelle pour un prélèvement ou en quantité suffisante pour prélèvements multiples,
◆ un réactif pour le dosage des D-dimères, et
◆ éventuellement un tampon de dilution des échantillons tel qu'un tampon Phosphate pH 7,4 contenant 0,1% de serum Bovine albumine et 0,05% de Tween 20.

Les plasmas contrôles positifs et négatifs sont avantageusement sous forme lyophilisée.

Le PA-Fb sp préférentiellement utilisé est le tPA.

Les D-dimères sont dosés par exemple avec un réactif pour méthode de type ELISA, tel que l'ASSERACHROM D-Di, ou un réactif pour un test sensible d'agglutination de particules de latex tel que le STA LIATEST D-Di, tous deux commercialisés par Diagnostica Stago.

Les exemples ci-après illustrent la présente invention.

### Exemple n° 1 :

### Choix de la concentration de thrombine utilisée pour l'obtention d'un plasma contrôle positif renfermant de la fibrine soluble :

Le plasma contrôle positif est préparé selon le protocole suivant :

| | |
|---|---|
| Plasma normal | 300 µl |
| Thrombine humaine (Stago réf. 00896) à 2 U/ml | 30 µl |
| Incubation de 2 min. à la température du laboratoire. | |
| Hirudine (Knoll) | 100 µg/ml (concentration finale) |
| ou Héparine (Choay) | 500 u/ml (concentration finale) |

Vérifier :
- qu'il n'y a pas eu de formation de caillot dans le tube.
- qu'un test de détection de la fibrine soluble disponible dans le commerce soit positif (ex. FS test du laboratoire Stago).

Deux possibilités présentées dans le tableau I peuvent être retenues.

**Tableau I**

| I-A : Tube 2 retenu. | | | | | |
|---|---|---|---|---|---|
| Nos des tubes | | 1 | 2 | 3 | 4 |
| Plasma normal citraté | | 300 µl | 300 µl | 300 µl | 300 µl |
| Thrombine | | 30 µl à 4 U/ml | 30 µl à 2 U/ml | 30 µl à 1 U/ml | 30 µl à 0,5 U/ml |
| | | Incubation de 2 minutes à la température ambiante | | | |
| Présence d'un caillot | | + | - | - | - |
| Héparine ou hirudine | | 500 unités 100 µg | 500 unités 100 µg | 500 unités 100 µg | 500 unités 100 µg |
| I- B : Tube 3 retenu | | | | | |
| Nos des tubes | | 1 | 2 | 3 | 4 |
| Plasma normal citraté | | 300 µl | 300 µl | 300 µl | 300 µl |
| Thrombine | | 30 µl à 4 U/ml | 30 µl à 2 U/ml | 30 µl à 1 U/ml | 30 µl à 0,5 U/ml |
| | | Incubation de 10-15 minutes à la température ambiante | | | |
| Présence d'un caillot | | + | + | - | - |
| Héparine ou hirudine | | 500 unités 100 µg | 500 unités 100 µg | 500 unités 100 µg | 500 unités 100 µg |

### Exemple n° 2 :

### Détermination de la quantité de PA Fb sp à utiliser dans des conditions d'incubation définies.

Pour réaliser la méthode de l'invention, la quantité d'activateur à ajouter à l'échantillon doit être telle qu'elle induise une génération des D-dimères importante dans le plasma contrôle positif, tel qu'obtenu dans l'exemple n° 1, et une génération des D-dimères non significative dans un plasma contrôle négatif (témoin non traité par la thrombine).

Une incubation des plasmas témoins et des plasmas contrôles positifs (n = 21) a donc été réalisée avec différentes doses de PA Fb sp pendant 15 minutes à 37° C. A la fin du temps d'incubation, les D-dimères sont déterminés par Liatest ou par Elisa rapide (D-Di Stago) (incubation de 15 minutes à 37° C avec l'anticorps de capture et de 15 minutes à 37° C avec l'anticorps de révélation).

Les résultats présentés dans la tableau I ont été obtenus avec le test ELISA.

Des résultats sensiblement analogues ont été obtenus avec le Liatest (n = 5).

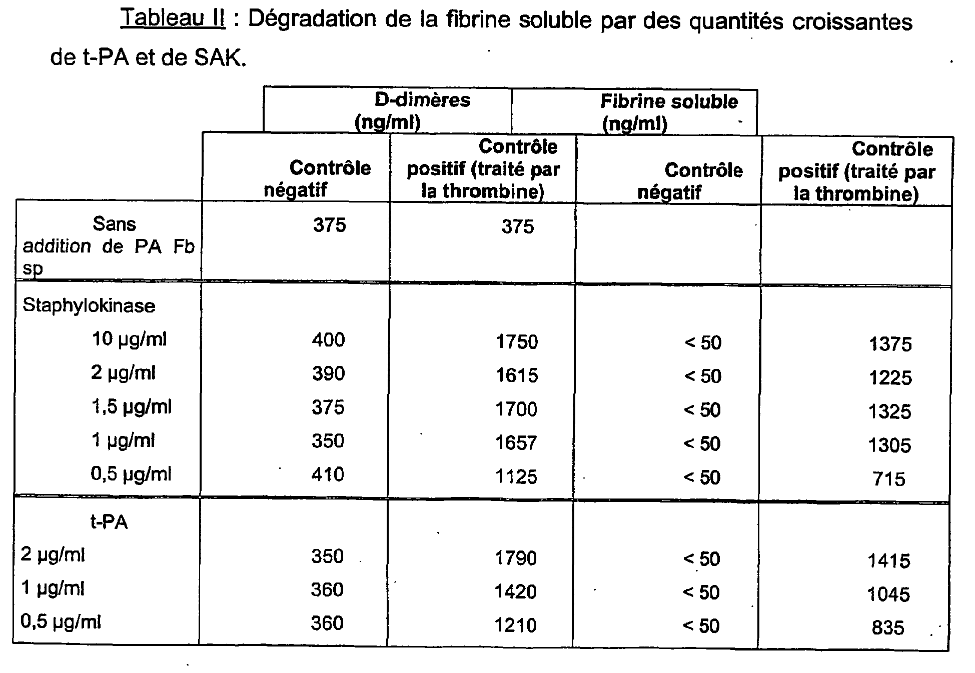

La dose de PA Fb sp choisie est celle qui induit :
- une augmentation < 300 ng/ml dans les plasmas contrôles non traités (témoins négatifs),
- l'augmentation la plus importante dans les plasmas contrôles positifs.

De ces résultats, il ressort que les concentrations finales préférées de PA Fb sp à utiliser sont :
- 2 µg/ml pour le t-PA : dans ces conditions, la dose de t-PA susceptible d'être neutralisée par les PAI est négligeable,
- 10 µg/ml pour la SAK (des doses inférieures de SAK ont induit une mauvaise dégradabilité de la fibrine soluble chez certains malades ou certains contrôles positifs, vraisemblablement du fait de la présence d'anti-staphylokinase dans le prélèvement, anti-staphylokinase qui peuvent apparaître, suite à une infection par des staphylocoques).

### Exemple n° 3 :

### Résultats obtenus chez des volontaires sains et chez des malades présentant une suspicion d'activation de la coagulation (du fait d'une augmentation des D-dimères).

La méthode est réalisée sur des échantillons de plasma, selon le protocole indiqué ci-dessus : incubation des plasmas pendant 15 minutes à 37° C en présence de tPA (2 µg/ml) ou de SAK (10 µg/ml).

Les D-dimères générés sont dosés par un test ELISA tel que décrit précédemment.

### A. Résultats obtenus chez le volontaire sain.

**Tableau III**

| | **F.S. (ng/ml)** |
|---|---|
| **Plasmas contrôles + t-PA (n = 21)** | **147 ± 100 ng/ml** |
| **Plasmas contrôles + IIa + t-PA (n=21)** | **2128 ± 1219 ng/ml** (valeurs extrêmes : 742 - 3660) |
| **Plasmas contrôles + SAK (n = 11)** | **64** ± **82 ng/ml** (valeurs extrêmes : 0-215) |
| **Plasmas contrôles + IIa + SAK (n=11)** | **1700** ± **1880 ng/ml** (valeurs extrêmes : 250 - 5000) |

### B. Résultats obtenus chez les malades présentant un taux des D-dimères élevé.

Expérience réalisée par Elisa rapide).

**Tableau IV :**

| Exemples trouvés dans notre étude : | | | |
|---|---|---|---|
| | **Taux de D-dimères après addition de t-PA (ng/ml).** | **Taux de D-dimères avant addiction de t-PA (ng/ml)** | **Taux de fibrine soluble (ng/ml)** |
| Malade du Groupe 1 | 5420 | 510 | 4910 |
| Malade du Groupe 2 | 1316 | 1234 | 82 |
| Malade du Groupe 3 | 30162 | 20699 | 9463 |

### CONCLUSIONS :

La méthode selon l'invention permet de séparer les malades en trois groupes, en fonction de leur teneurs plasmatiques en fibrine soluble et en D-dimères. Les caractéristiques de chacun de ces trois groupes sont résumées dans le tableau V ci-dessous :

| | **Fibrine soluble (ng/ml)** | **D-dimères (ng/ml)** | **Conclusion** |
|---|---|---|---|
| Groupe 1 | > 500 | +/- | Formation d'un caillot qui n'est pas encore dégradé. |
| Groupe 2 | < 300 | +/+++ | Présence d'un thrombus, mais **coagulation arrêtée.** |
| Groupe 3 | > 500 | +++ | **Coagulation** persiste, associée à une **dégradation** du caillot. |

Comme cela l'a été indiqué précédemment, la méthode de l'invention permet ainsi non seulement de suivre l'évolution d'un processus d'activation de la coagulation mais également d'évaluer l'efficacité d'une thérapeutique anticoagulante, et d'apprécier si à l'arrêt de la thérapeutique il y a de nouveau activation de la coagulation.

**Groupe 1** : activation de la coagulation précoce avec augmentation de la fibrine soluble, sans augmentation des D-dimères.

**Groupe 2** : malades pour lesquels l'activation de la coagulation est stoppée (thérapeutique efficace), mais le thrombus déjà formé continue à se dégrader (teneur en fibrine soluble normale, taux des D-dimères augmenté).

**Groupe 3** : malades présentant une activation de la coagulation avec dégradation du caillot in vivo (augmentation simultanée de la fibrine soluble et des D-dimères) : thérapeutique pas assez efficace.

### Exemple n° 4 :

### Récapitulatif de la technique utilisée :

Réactifs :
   Tampon pH 7,4
   Thrombine humaine purifiée (Stago Réf. 00896)
   t-PA (Boehringer)
   Aprotinine. Garder la solution à 4°C.
   Kit de D-Dimères.

La méthode utilisée est résumée dans le tableau VI.

**Tableau VI : Protocole utilisé pour le dosage de la fibrine soluble par génération de D-Dimères.**

| | |
|---|---|
| Plasma | 200 |
| t-PA (20 µg/ml) (conc. finale 2 µg/ml) | 20 |
| Maintenir 15' à 37° C | |
| Aprotinine | 20 |
| Diluer l'échantillon en fonction du taux de D-dimères de base (1/20 - 1/1000) | |
| Plaque Asserachrom D-Di | |
| Echantillon dilué | 200 |
| Couvrir les puits et maintenir 15' à 37° C. | |
| Laver 3 fois | |
| Anti-fragment D humain marqué à la peroxydase | 200 |
| Couvrir les puits et maintenir 15' à 37° C | |
| Laver 3 fois | |
| Substrat OPD/H₂O₂ | 200 |
| Attendre 3 minutes très exactement pour chaque échantillon, puis ajouter : | |
| soit H₂SO₄ 3M | 50 |
| soit Hcl 1M | 100 |
| Mesurer l'absorbance à 492 nm. | |

### BIBLIOGRAPHIE :

1. GODAL H.C., ABILDGAARD U. : "Gelation of soluble fibrin in plasma by ethanol". Scand. J. Haematol., 3, 342-350, 1966.
2. BREEN F.A., TULLIS J.L. : "Ethanol gelation : a rapid screening test for intravascular coagulation". Ann. Intern. Med., 69, 1197-1206, 1968.
3. LIPINSKI B., WOROWSKI K. : "Detection of soluble fibrin monomer complexes in blood by means of protamine sulphate test". Thromb. Diath. Haemorrh., 20, 44-49, 1968.
4. SEAMAN A.J. : "The recognition of intravascular clotting. The plasma protamine paracoagulation test". Arch. Intern. Med., 125, 1016-1021, 1970.
5. LATALLO Z.S., WEGRZYNOWICZ S., BUDZUNKI A.Z. : "Effect of protamine sulphate on the solubility of fibrinogen, its derivatives and other plasma proteins". Scand. J. Haematol., suppl., 13, 151-162, 1971.
6. MUSUMECI V. : "Ethanol gelation test and protamine sulphate test in diagnosis of intravascular coagulation". Scand. J. Haematol., suppl., 13, 197-202, 1971.
7. NIEWIAROWSKI S., GUREWICH V. : "Laboratory identification of coagulation. The serial dilution protamine sulfate test for the détection of fibrin monomer and fibrin degradation products". J. Lab. Clin. Med., 77, 665-676, 1971.
8. KIDDER W.R., LOGAN L.J., RAPAPORT S.I., PATCH M.J. : "The plasma protamine paracoagulation test - Clinical and laboratory evaluation". A.J.C.P., 58, 675-686, 1972.
9. GUREWICH V., LIPINSKI B., LIPINSKA I. : "A comparative study of précipitation and paracoagulation by protamine sulphate and ethanol gelation tests". Thromb. Res., 2, 539-556, 1973.
10. GERRITS W.B.J., PRAKKE E.M., VAN DER MEER J., VREEKEN J. : "Causes of a negative ethanol gelation test in diffuse intravascular coagulation". Thromb. Diath. Haemorrh., 31, 299-308, 1974.
11. LARGO R., HELLER V., STRAUB P.W. : "Detection of soluble intermediates of the fibrinogen-fibrin conversion using erythrocytes coated with fibrin monomers". Blood, 47, 991-1002, 1976.
12. SORIA J., SORIA C., DE RODRIGUEZ S., HORELLOU M.H., SAMAMA M., BILSKI-PASQUIER G. : "Recherche des complexes solubles de fibrine par un test d'hémagglutination - Applications cliniques". Presse Méd., 6, 43, 4045-4048, 1977.
13. DEMPFLE CE., DOLLMAN M., LILL H., PUZZOVIO D., DESSAUER A., HEENE DL. : "Binding of a new monoclonal antibody against N-terminal heptapeptide of fibrin alpha-chain to fibrin polymerization site "A" : effects of fibrinogen and fibrinogen derivatives, and pretreatment of samples with NaSCN. Blood Coagul. Fibrinolysis, 4, 79-86, 1993.
14. SOE G., KOHNO I., INUZUKA K., ITOH Y., MATSUDA M. : "A monoclonal antibody that recognizes a neo-antigen exposed in the E domain of fibrin monomer complexed with fibrinogen or its derivatives : its application to the measurement of soluble fibrin in plasma. Blood, 88, 2109-2117, 1996.
15. GUREWICH V., HYDE E., LIPINSKI B. : "The resistance of fibrinogen and soluble fibrin monomer in blood to degradation by a potent plasminogen activator derived from cadaver limbs. Blood, 46, 555-565, 1975.
16. CANNON CP, GIBSON CM, MCCABE CH, ADGEY AA, SCHWEIGER MJ, SEQUEIRA RF, GROLLIER G, GIUGLIANO RP, FREY M, MUELLER HS, STEINGART RM, WEAVER WD, VAN DE WERF F, BRAUNWALD E. : "TNK-tissue plasminogen activator compared with front-loaded altephase in acute myocardial infarction results of the TIMI 10B trial". Thrombolysis in Myocardial Infarction (TIMI) 10B Investigators, Circulation 98 (25), 2805-14, 1998.
17. BRINGMANN P, GRUBER D, LIESE A, TOSCHI, L, KRATZCHMAR J, SCHLEUNING WD, DONNER P. : "Structural features mediating fibrin selectivity of vampirebat plasminogen activators". J Biol Chem, 270, 25596-603, 1995.
18. COLLEN D. : "Staphylokinase : a potent, uniquely fibrin-selective thrombolytic agent". Nat Med, 4 - 279-84, 1998.
19. SAKHAROV DV, BARRERTT-BERGSHOEFF M, HEKKENBERG RT, RIJKEN DC. : "Fibrin-specificity of a plasminogen activator affects the efficiency of fibrinolysis and responsiveness to ultrasound : comparison of nine plasminogen activators in vitro". Thromb Haemos, 81, 605-12, 1999.
20. COLLEN D, BERNAERTS R, DECLERCK, P, DE COCK F, DEMARSIN E, JENNE S. LAROCHE Y, LIJNEN HR, SILENCE K, VERSTREKEN M. : "Recombinant staphylokinase variants with altered immunoreactivity. I : Construction and characterization". Circulation, 94, 197-206, 1996.

## Revendications

1. Méthode de dosage de la fibrine soluble dans un échantillon, dans laquelle on met ledit échantillon en présence d'un activateur de plasminogène de forte spécificité pour la fibrine soluble (PA-Fb sp) spécifique de la fibrine dans les conditions d'utilisation de l'activateur sélectionnées pour éviter la dégradation du fibrinogène plasmatique circulant et l'on mesure la teneur de l'échantillon en fibrine soluble, en mesurant la différence entre le taux de produits de dégradation spécifiques de la fibrine obtenus après dégradation de la fibrine soluble par incubation avec le PA-Fb sp et le taux de base des produits de dégradation de la fibrine, déterminé sur ledit échantillon avant sa mis en présence avec le PA-Fb sp.

2. Méthode selon la revendication 1, **caractérisée en ce que** les produits de dégradation de la fibrine mesurés sont les D-dimères.

3. Méthode selon la revendication 1, **caractérisée en ce que** le PA-Fb sp est choisi au sein d'un groupe constitué par : le tPA ou ses dérivés, le VPA ou ses dérivés, la staphylokinase ou un de ses mutants.

4. Méthode selon la revendication 1, **caractérisée en ce que** le PA-Fb sp est le tPA ou la staphylokinase.

5. Méthode selon la revendication 1, **caractérisée en ce que** l'échantillon sanguin est du plasma.

6. Méthode selon la revendication 1, **caractérisée en ce que** les produits de dégradation de la fibrine sont dosés selon une méthode de type ELISA, ou LIATEST.

7. Méthode selon l'une quelconque des revendications 1 à 5, **caractérisée en ce que** l'échantillon à tester est incubé pendant 15 minutes à 37° C en présence de PA-Fb sp.

8. Méthode selon l'une quelconque des revendications 1 à 6, **caractérisée en ce que** le PA-Fb sp est le t-PA utilisé dans une gamme de concentrations finales comprise entre 1 et 2,5 µg/ml.

9. Méthode selon l'une quelconque des revendications 1 à 6, **caractérisée en ce que** le PA-Fb sp sst le 1PA à une concentration finale de 2 µg/ml d'éhantillon.

10. Méthode selon l'une quelconque des revendications 1 à 6, **caractérisée en ce que** la PA-FB sp est choisie parmi la Staphylokinase, la battPA, la VPA, la DSPAs, la FEKP, IEKP et la KP.

11. Méthode selon l'une quelconque des revendication 1 à 6. **caractérisée en ce que** le PA-Fb sp est la staphylokinase à une concentration finale de 10 µg/ml d'échantillon.

12. Méthode selon l'une quelconque des revendications 1 à 10, **caractérisée en ce que** les taux de produits de dégradation de la fibrine soluble résultant de l'activation du PA-Fb, sp sont appréciés par rapport à un témoin positif préparé à partir d'un plasma normal traité par un activateur de la coagulation puis par un inhibiteur dudit activateur pour éviter la formation d'un caillot.

13. Méthode selon l'une des revendications 1 à 12, **caractérisée en ce que** le témoin positif est préparé à partir d'un plasma normal traité avec de la thrombine de façon à induire une activation de la coagulation in vitro puis avec de l'hirudine ou de l'héparine, pour éviter la formation d'un caillot.

14. Utilisation de la méthode selon l'une quelconque des revendications 1 à 13, pour suivre l'évolution d'un processus d'activation de la coagulation et évaluer l'efficacité d'une thérapeutique anti-coagulante.

## Claims

1. A method for assaying soluble fibrin in a sample, in which said sample is brought into the presence of a plasminogen activator with a high specificity for soluble fibrin (PA-Fb sp), which is specific for fibrin in conditions for use of the activator such that degradation of the circulating plasmatic fibrinogen is avoided, and the soluble fibrin count in the sample is measured by measuring the difference between the count of fibrin degradation specific products obtained after degrading soluble fibrin by incubation with PA-Fb sp and the base count of fibrin degradation products determined for said sample before bringing it into the presence of PA-Fb sp.

2. A method according to claim 1, **characterized in that** the fibrin degradation products that are measured are D-dimers.

3. A method according to claim 1, **characterized in that** the PA Fb sp is selected from the group formed by: tPA and its derivatives, VPA or its derivatives, and staphylokinase or one of its mutants.

4. A method according to claim 1, **characterized in that** the PA Fb sp is t-PA or staphylokinase.

5. A method according to claim 1, **characterized in that** the blood sample is plasma.

6. A method according to claim 1, **characterized in that** the fibrin degradation products are assayed using an ELISA or LIATEST type method.

7. A method according to any one of claims 1 to 5, **characterized in that** the test sample is incubated for 15 minutes at 37°C in the presence of PA Fb sp.

8. A method according to any one of claims 1 to 6, **characterized in that** the PA Fb sp is tPA used in a final concentration in the range of 1 to 2.5 µg/ml.

9. A method according to any one of claims 1 to 6, **characterized in that** the PA Fb sp is tPA in a final concentration in the range of 2 µg/ml of the sample.

10. A method according to any one of claims 1 to 6, **characterized in that** the PA Fb sp is selected from staphylokinase, bat-tPA, VPA, DSPAs, FEKP, EKP and KP.

11. A method according to any one of claims 1 to 6, **characterized in that** the PA Fb sp is staphylokinase in a final concentration of 10 µg/ml of sample.

12. A method according to any one of claims 1 to 10, **characterized in that** the degradation product counts in the soluble fibrin resulting from PA Fb sp activation are determined with respect to a positive control prepared from a normal plasma treated by a coagulation activator then by an inhibitor of said activator to avoid the formation of a clot.

13. A method according to to any one of claims 1 to 12, **characterized in that** the positive control is prepared from a normal plasma treated with thrombin so as to induce in vitro coagulation activation then with hirudin or heparin, to avoid the formation of a clot.

14. Use of the method of any one of claims 1 to 13, to monitor the change in a coagulation activation process and to evaluate the efficacy of an anti-coagulant drug.

## Patentansprüche

1. Verfahren zur Bestimmung von in einer Probe löslichem Fibrin, bei welchem die Probe einem vorhandenen Plasminogen-Aktivator mit hoher Spezifizität für lösliches Fibrin (PA-Fb sp) ausgesetzt wird, der für das Fibrin unter Bedingungen der Verwendung des ausgewählten Aktivators spezifisch ist, um den Abbau des zirkulierenden plasmatischen Fibrins zu vermeiden, und der Gehalt an löslichem Fibrin in der Probe gemessen wird, indem der Unterschied zwischen dem Spiegel an für das Fibrin spezifischen Abbauprodukten, der nach Abbau des löslichen Fibrins durch Inkubieren mit dem PA-Fb sp erhalten wurde, und dem Grundspiegel an Abbauprodukten des Fibrins, der für die Probe bestimmt wurde, bevor diese in Gegenwart mit PA-Fb sp gebracht wurde, gemessen wird.

2. Verfahren gemäß Anspruch 1, **dadurch gekennzeichnet, dass** die gemessenen Abbauprodukte des Fibrins D-Dimere sind.

3. Verfahren gemäß Anspruch 1, **dadurch gekennzeichnet, dass** der PA-Fb sp ausgewählt ist aus einer Gruppe bestehend aus: tPA oder dessen Derivaten, VPA oder dessen Derivaten, Staphylokinase oder einer ihrer Mutanten.

4. Verfahren gemäß Anspruch 1, **dadurch gekennzeichnet, dass** der PA-Fb sp tPA oder Staphylokinase ist.

5. Verfahren gemäß Anspruch 1, **dadurch gekennzeichnet, dass** die Blutprobe Plasma ist.

6. Verfahren gemäß Anspruch 1, **dadurch gekennzeichnet, dass** die Abbauprodukte des Fibrins mit einem Verfahren der Art ELISA oder LIATEST bestimmt werden.

7. Verfahren gemäß einem der Ansprüche 1 bis 5, **dadurch gekennzeichnet, dass** die zu untersuchende Probe für 15 Minuten bei 37 °C in Gegenwart von PA-Fb sp inkubiert wird.

8. Verfahren gemäß einem der Ansprüche 1 bis 6, **dadurch gekennzeichnet, dass** der PA-Fb sp t-PA ist, der in einem Endkonzentrationsbereich zwischen 1 und 2,5 µg/ml verwendet wird.

9. Verfahren gemäß einem der Ansprüche 1 bis 6, **dadurch gekennzeichnet, dass** der PA-Fb sp t-PA in einer Endkonzentration von 2 µg/ml Probe ist.

10. Verfahren gemäß einem der Ansprüche 1 bis 6, **dadurch gekennzeichnet, dass** der PA-Fb sp ausgewählt ist aus Staphylokinase, bat-tPA, VPA, DSPAs, FEKP, EKP und KP.

11. Verfahren gemäß einem der Ansprüche 1 bis 6, **dadurch gekennzeichnet, dass** der PA-Fb sp Staphylokinase in einer Endkonzentration von 10 µg/ml Probe ist.

12. Verfahren gemäß einem der Ansprüche 1 bis 10, **dadurch gekennzeichnet, dass** die Spiegel an Abbauprodukten des löslichen Fibrins, die sich aus der Aktivierung des PA-Fb sp ergeben, bezogen auf eine Positivkontrolle bewertet werden, die aus normalem Plasma hergestellt wird, das mit einem Koagulationsaktivator, anschließend mit einem Inhibitor des Aktivators, um die Bildung eines Gerinnsels zu vermeiden, behandelt wurde.

13. Verfahren gemäß einem der Ansprüche 1 bis 12, **dadurch gekennzeichnet, dass** die Positivkontrolle aus einem normalen Plasma hergestellt wird, das mit Thrombin, um so eine Aktivierung der Koagulation in vitro einzuleiten, anschließend mit Hirudin oder Heparin, um die Bildung eines Gerinnsels zu vermeiden, behandelt wurde.

14. Anwendung des Verfahrens gemäß einem der Ansprüche 1 bis 13 zur Verfolgung der des Fortschreitens eines Vorgangs zur Aktivierung der Koagulation und Bewertung der Wirksamkeit einer Anti-Koagulationstherapie.
